# EUROPEAN PATENT SPECIFICATION

(11) **EP 2 189 150 B1**
(45) Date of publication and mention of the grant of the patent: **29.01.2014**
(21) Application number: 09176948.9
(22) Date of filing: 24.11.2009
(51) Int. Cl.: A61K 8/36, A61Q 1/12, A61K 8/58, A61K 8/891

(54) **Process for preparing a compacted cosmetic product**
Verfahren zur Herstellung eines kompaktierten Kosmetikproduktes
Procédé de préparation d'un produit cosmétique compact

(30) Priority: 25.11.2008 IT MI20082101
(43) Date of publication of application: 26.05.2010
(73) Proprietor: CHROMAVIS S.p.A., 20122 Milan (IT)
(72) Inventor: Brambilla, Andreina, 20060, Pozzuolo Martesana (MI) (IT)
(74) Representative: Vanosi, Adelio Valeriano

(56) References cited:
- EP-A2- 0 976 388
- US-A- 5 496 544
- US-A- 6 001 373
- US-A1- 2003 133 895
- US-A1- 2005 191 329

## Description

The present invention relates to a process for preparing cosmetic product in powder form, such as eye shadow, all-over powder, face powder, blusher or bronzing powder.

Traditional products in powder form are prepared by dispersing a binding phase in a mixture of powders for cosmetic use. This dispersion is prepared using a mixing apparatus, commonly in the form of a propeller and/or blade mixer, rotating at high speed (2500-3500 r.p.m.) The powder is then compacted and placed in a base.

This process results in the creation of products having a dry feel on application, this being uncomfortable for persons with a dry skin, and moreover the products are of low elasticity and ductility from the production viewpoint. Elasticity and ductility are very important when significant reliefs are required on the product surface external to the base. Moreover, known products tend to crumble when subjected to impact.

In addition, traditional compact powder products present a tendentially opaque appearance and only slightly pearly, even when large quantities of pearlescent elements are present. In this case they are more friable and tend to crumble with considerable ease.

An object of the present invention is to provide process for preparing a cosmetic product presenting a highly pearly appearance, improved ductility and elasticity, better adherence to the skin and greater comfort when used on dry skin.

This and other objects are attained by a process for preparing cosmetic product in accordance with the technical teachings of the accompanying claims.

Further characteristics and advantages of the invention will be apparent from the description of a preferred but non-exclusive embodiment of the preparation method, described by way of non-limiting example hereinafter.

The method for producing the cosmetic product of the present invention consists of the following steps:
a. preparing a mixture of powders for cosmetic use,
b. obtaining an emulsion by emulsifying at least one volatile silicone, at least one non-volatile silicone, at least one water/silicone emulsifier and water,
c. mixing the mixture of powders and the emulsion by kneading them together in a kneading machine,
d. drying the mixture obtained under the preceding point to facilitate loss of volatile silicone and water, until a residue is obtained containing less than 2% by weight of volatile silicone and less than 2% by weight of water,
e. sieving the mix obtained, and
f. compacting it.

The mixture of powders for cosmetic use is preferably prepared using a high speed mixer; the pearls are added only at the end of the process and blown within the mill for a very short time (of the order of a few seconds). The term "powders for cosmetic use" comprises inert and non-inert powders, such as:
Talc
Mica
Bismuth oxychloride (CI77163)
Iron oxides (CI77491-2-9)
Ultramarine blue (CI77007)
Manganese violet (CI77742)
Chromium hydroxide (CI77289)
Chromium oxide (CI77288)
Ferric ammonium ferrocyanide (CI77288)
Titanium dioxide (CI77891)
D&C Red7 CA lake
D&C Red19 Al Lake
D&C Red6 Ba Lake
D&C Red3 Al Lake
D&C Red9 BA Lake
D&C Red21 Al Lake
D&C Yellow5 Al Lake
D&C Red30 Al Lake
D&C Yellow10 Al Lake
D&C Red27 Al Lake
D&C Yellow5 Al Lake
D&C Orange5
FD&C Yellow6 Al Lake
FD&C Blue1 Al Lake
D&C Red36 Al Lake
Carmine (CI75470)
Fluorphlogopite (Synthetic mica)
Boron nitride
Calcium aluminium borosilicate
Magnesium aluminium borosilicate

In the aforegoing list the definition "Cl" in parentheses represents the Colour Index reference. CTFA names (organic lakes) are also present.

In particular, the powder mixture presents a very high quantity, between 20 and 70%, of pearlescent pigments (usually in the form of mica coated with titanium oxide).

The water/silicone (W/S) emulsion is prepared using a suitable turboemulsifier in accordance with the known art. For the W/S emulsion, silicones pertaining to the silane and siloxane family are used.

They are mixed in the following calculated weight percentages:
- volatile silicones acting as solvent (from 1% to 22%, preferably 15%)
- non-volatile silicones acting as binder/emollient (from 5% to 40%, preferably 18%)
- emulsifying silicones (from 0.3% to 5%, preferably 0.7%)
- water acting as solvent (from 5.5% to 97.025%, preferably 70.65%)
- preferably at least one gelling agent (from 0.2% to 5%, preferably 3%)

In this context, volatile silicones are those characterised by a viscosity index between 0.65 and 10 cSt. Some usable volatile silicones are for example those comprised in the group: Cyclomethicone, Cyclopentasiloxane, Ciclohexasiloxane, Disiloxane, Trisiloxane, Methyl Trimethicone, Dimethicone with said viscosity index.

Volatile silicones are well known in the cosmetic field. A description of various volatile silicones can be found in Tood et al. "Volatile Silicone Fluids for Cosmetics", 91 Cosmetics and Toiletries (1976).

More specifically in this context, non-volatile silicones are those characterised by a viscosity index between 10 and 5000 cSt. Some usable non-volatile silicones are for example those comprised in the group: Dimethicones, Alkyl Siloxane, Dimethiconol, Dimethicone Crosspolymer, Vinyl Dimethicone/Methicone Silsequioxane Crosspolymer, Phenyl Trimethicone, Diphenyl Trimethicone, Methicone, Trimethylsiloxysilicate, Triethoxycaprylilsilane, silicone gums, and their combinations with said viscosity index.

Emulsifying silicones are those which in their molecule contain functional groups able to form a bond with water and which preferably have a viscosity index between 100 and 5000 cSt. Examples of water/silicone emulsifiers are for example: PEG dimethicone, Dimethicones Copolyol, PPG/PEG Dimethicones, ABn Dimethicones Copolymer, and combinations of this latter.

All the cSt values are calculated at 25°C.

Preferably an organic salt can be used as the gelling agent, for example Al/Mg hydroxystearate, zinc stearate, magnesium myristate, or an inorganic salt such as NaCl or MgSO4.

Hence the process for preparing the cosmetic product comprises the succession of aforedescribed steps where in particular the mix is obtained by mixing the powders for cosmetic use with the W/S emulsion. This mixing takes place using a low speed (5-500 r.p.m.) kneading machine. The mixing ratio between the two aforedescribed parts can lie within a range described in the table below:

| **Description** | **% Ideal** | **% Minimum** | **% Maximum** |
|---|---|---|---|
| **Mix of "powders for cosmetic use"** | 50 | 20 | 80 |
| **W/S Emulsion** | 50 | 80 | 20 |

The kneaded mix obtained can be dried in an oven and/or under vacuum, at a temperature within the range given in the table below, for a time between 8 and 96 hours. Essentially, drying is protracted (as can be seen hereinafter) until both the aqueous component and the volatile silicone component have almost completely evaporated and less than 4% by weight (<2% of water, <2% of volatile silicone) remain in the intermediate product. The percentage of water and volatile silicone that is present in the intermediate product after that it is dried is the same that it is present in the final product, as no more water or volatile silicones are added during the process.

| **Temperature** | **Ideal Value** | **Minimum Value** | **Maximum Valee** |
|---|---|---|---|
| **Unit of measurement Degrees Celsius C°** | 50C° | 20C° | 100C° |

After extracting the solvents or volatile components the mix is sieved, preferably using a mechanical vibrating screen over metal meshes having a mesh range between 4.57 and 200, the main purpose being to obtain a uniform product particle size.

The product of the present invention is particularly easy to sieve as the amalgam is perfectly homogeneous with no need for several screening passes or new mill dispersions as takes place in traditional compacts.

Finally the powder obtained after the aforedescribed steps is compacted, preferably using a compacting machine, at pressures between 10 and 100 bar, for times between 10 and 90 tenths of a second, for a number of pressings between 1 and 10 pressings.

The product is compacted into a seat commonly known as a "base" which can be formed of various materials, such as PE, aluminium, tinplate, etc. This procedure enables a compacted powder product to be obtained with an extreme lustre, achieved by being able to use high percentages of pearlescent pigments (20-70% by weight), commonly not usable in a traditional product, and by virtue of the minimum mechanical impact of the kneading machine on these pearlescent pigments.

In this respect, a strong mechanical impact would cause detachment of the titanium or iron oxide coating from the mica with which the pearlescent pigments are formed.

Both their feel and application are slidable, velvety, and not greasy as happens with traditional products having a similar degree of lustre.

Their elasticity is very high, as demonstrated by drop tests carried out on the finished product. It does not fracture as does a traditional powder product when subjected to a drop for example of 50 cm, but remains as a single piece which at most tends to lift from the seat (base) within which it is homogeneously located.

Moreover, the powder is compacted at pressures which are much less than those traditionally employed. This considerably reduces processing times, so increasing productivity.

Preferably in the final cosmetic product said volatile silicone is present in a percentage of 1.2%, and/or non-volatile silicone is present in a percentage between 7% and 18%, preferably 14%, and/or said water/silicone emulsifier is present in a percentage between 0.3% and 5%, preferably 0.6%.

During the process the kneaded mix is dried until a residue is obtained containing less that 2% of volatile silicone and less than 2% of water, and preferably the mixture of powders is kneaded with said emulsion in a percentage varying from 20 to 80%, preferably 50%.

### FORMULATION EXAMPLES

In a product preparation example, a mixture of powders for cosmetic use is prepared having the following composition by weight:

### Powder mixture

| **PRODUCT** | **PERCENTAGE** |
|---|---|
| Sodium Dehydroacetate | 0.3 |
| Sorbic Acid | 0.4 |
| Magnesium Myristate | 3 |
| Zinc Laurate | 3 |
| Zinc Stearate | 0.9 |
| Talc | 30.4 |
| Silica | 2 |
| Mica/pearls | 40 |
| Pigment | 20 |

This mixture is prepared in a standard mill The pearlescent elements are added at the end of the process to maintain their properties unaltered.

A water/silicone emulsion is then prepared using a turboemulsifier.

The emulsion comprises the following components in the listed percentages by weight:

### W/S emulsion

| **PRODUCT** | **PERCENTAGE** |
|---|---|
| Water | 62.14 |
| Cyclopentasiloxane | 7.14 |
| Cyclomethicone | 4 |
| Cyclohexasiloxane | 3.84 |
| Dimethicone | 11.44 |
| Phenyl Trimethicone | 5 |
| Trimethylsiloxysilicate | 1 |
| Cetyl PEG/PPG-10/1 Dimethicone | 0.24 |
| Polyglyceryl-4 Isostearate | 0.24 |
| Hexyl Laurate | 0.26 |
| Sodium Chloride | 0.5 |
| Aluminum/Magnesium Hydroxy Stearate | 1 |
| Polysilicone-13 | 2 |
| Phenoxyethanol | 1.2 |

The powder mixture and emulsion are then kneaded together by a Conti PL760EV kneading machine at 300 r.p.m. for a time which can vary indicatively from 5 to 10 minutes.

In this manner the following kneaded mix is obtained:

### Mix:

| **PRODUCT** | **PERCENTAGE** |
|---|---|
| Water | 31,07 |
| Cyclopentasiloxane | 3,57 |
| Cyclomethicone | 2 |
| Cyclohexasiloxane | 1.92 |
| Dimethicone | 5.72 |
| Phenyl Trimethicone | 2.5 |
| Trimethylsiloxysilicate | 0.5 |
| Cetyl PEG/PPG-10/1 Dimethicone | 0.12 |
| Polyglyceryl-4 Isostearate | 0.12 |
| Hexyl Laurate | 0.13 |
| Sodium Chloride | 0.25 |
| Aluminum/Magnesium Hydroxy Stearate | 0.5 |
| Polysilicone-13 | 1 |
| Sodium Dehydroacetate | 0.15 |
| Sorbic Acid | 0.2 |
| Phenoxyethanol | 0.6 |
| Magnesium Myristate | 1.5 |
| Zinc Laurate | 1.5 |
| Zinc Stearate | 0.45 |
| Talc | 15.2 |
| Silica | 1 |
| Mica/pearls | 20 |
| Pigment | 10 |

The mix is dried in an oven at atmospheric pressure, set at a temperature of 50°C for a time of about 24 hours or until the aqueous component in the product has been brought to about 0.3% and the volatile silicone component has been brought to about 1.2% such as to obtain a powder with the following composition:

### Finished product after drying:

| **PRODUCT** | **PERCENTAGE** |
|---|---|
| Water | 0.5 |
| Cyclopentasiloxane | 0.4 |
| Cyclomethicone | 0.4 |
| Cyclohexasiloxane | 0.4 |
| Dimethicone | 9.152 |
| Phenyl Trimethicone | 4 |
| Trimethylsiloxysilicate | 0.8 |
| Cetyl PEG/PPG-10/1 Dimethicone | 0.192 |
| Polyglyceryl-4 Isostearate | 0.192 |
| Hexyl Laurate | 0.208 |
| Sodium Chloride | 0.4 |
| Aluminum/Magnesium Hydroxy Stearate | 0.8 |
| Polysilicone-13 | 1.6 |
| Sodium Dehydroacetate | 0.24 |
| Sorbic Acid | 0.32 |
| Phenoxyethanol | 0.96 |
| Magnesium Myristate | 2.4 |
| Zinc Laurate | 2.4 |
| Zinc Stearate | 0.72 |
| Talc | 24.32 |
| Silica | 1.6 |
| Mica/pearls | 32 |
| Pigment | 16 |

On termination of the drying stage the product is sieved using a mechanical vibrating screen.

The sieved powder is then compacted into a base at a pressure of 15 bar for a time of 20 tenths of a second, for 5 successive pressings.

It should be noted that, as already stated, the aforestated percentages are expressed by weight, the viscosity being measured at 25°C.

The aforegoing example illustrates the use of three volatile silicones, however analogous results can be obtained by using only one silicone or more than three silicones.

The same is valid for the non-volatile silicones.

## Claims

1. A process for preparing a compacted product, comprising at least the steps of:
a. preparing a mixture of powders for cosmetic use;
b. **obtaining an emulsion by emulsifying** at least one volatile silicone, at least one non-volatile silicone, at least one water/silicone emulsifier and water,
**c. mixing the mixture of powders and the emulsion by kneading them together in a kneading machine,**
**d.** drying the mixture obtained under the preceding point to facilitate loss of volatile silicone and water, **until a residue is obtained containing less than 2% by weight of volatile silicone and less than 2% by weight of water,**
**e.** sieving the mix obtained, and
**f**.compacting it.

2. A process as claimed in one or more of the preceding claims, **characterised in that** the mixture of powders is kneaded with said emulsion in a percentage varying from 20 to 80%, preferably 50% **by weight**.

3. A process as claimed in one or more of the preceding claims, **characterised in that** said emulsion comprises between 1.0% and 22%, preferably 15% **by weight**, of said at least one volatile silicone.

4. A process as claimed in one or more of the preceding claims, **characterised in that** said emulsion comprises between 5% and 40%, preferably 15% **by weight**, of said at least one non-volatile silicone.

5. A process as claimed in one or more of the preceding claims, **characterised in that** said emulsion comprises between 0.3% and 5%, preferably 0.7% **by weight**, of said at least one emulsifying silicone.

6. A process as claimed in one or more of the preceding claims, **characterised in that** said emulsion comprises between 0.2% and 5%, preferably 3% **by weight**, of a gelling agent.

## Patentansprüche

1. Verfahren zur Herstellung eines kompaktierten Produkts, umfassend wenigstens die folgenden Schritte:
a. Herstellen einer Pulvermischung für kosmetische Verwendung;
b. Erhalten einer Emulsion durch Emulgieren wenigstens eines flüchtigen Silicons, wenigstens eines nichtflüchtigen Silicons, wenigstens eines Wasser/Silicon-Emulgators und Wasser,
c. Vermischen der Pulvermischung und der Emulsion durch Zusammenkneten derselben in einer Knetmaschine,
d. Trocknen der gemäß dem vorangehenden Punkt erhaltenen Mischung, um das Entweichen von flüchtigem Silicon und Wasser zu erleichtern, bis ein Rest erhalten wird, der weniger als 2 Gewichts-% flüchtiges Silicon und weniger als 2 Gewichts-% Wasser enthält,
e. Sieben der erhaltenen Mischung, und
f. Kompaktieren derselben.

2. Verfahren gemäß einem der vorangehenden Ansprüche, **dadurch gekennzeichnet, dass** die Pulvermischung mit der Emulsion in einem Prozentanteil, der von 20 bis 80%, vorzugsweise 50%, bezogen auf das Gewicht, variiert, geknetet wird.

3. Verfahren gemäß einem der vorangehenden Ansprüche, **dadurch gekennzeichnet, dass** die Emulsion zwischen 1,0% und 22%, vorzugsweise 15%, bezogen auf das Gewicht, des wenigstens einen flüchtigen Silicons umfasst.

4. Verfahren gemäß einem der vorangehenden Ansprüche, **dadurch gekennzeichnet, dass** die Emulsion zwischen 5% und 40%, vorzugsweise 15%, bezogen auf das Gewicht, des wenigstens einen nichtflüchtigen Silicons umfasst.

5. Verfahren gemäß einem der vorangehenden Ansprüche, **dadurch gekennzeichnet, dass** die Emulsion zwischen 0,3% und 5%, vorzugsweise 0,7%, bezogen auf das Gewicht, des wenigstens einen emulgierenden Silicons umfasst.

6. Verfahren gemäß einem der vorangehenden Ansprüche, **dadurch gekennzeichnet, dass** die Emulsion zwischen 0,2% und 5%, vorzugsweise 3%, bezogen auf das Gewicht, eines Gelierungsmittels umfasst.

## Revendications

1. Procédé de préparation d'un produit compacté, comprenant au moins les étapes consistant à :
a. préparer un mélange de poudres à usage cosmétique ;
b. obtenir une émulsion en émulsionnant au moins une silicone volatile, au moins une silicone non volatile, au moins un émulsifiant eau / silicone et de l'eau,
c. mélanger le mélange de poudres et l'émulsion en les malaxant ensemble dans un malaxeur,
d. sécher le mélange obtenu au point précédent pour faciliter la perte de silicone volatile et d'eau, jusqu'à obtention d'un résidu contenant moins de 2 % en poids de silicone volatile et moins de 2% en poids d'eau,
e. tamiser le mélange obtenu, et
f. le compacter.

2. Procédé selon l'une ou plusieurs des revendications précédentes, **caractérisé en ce que** le mélange de poudres est malaxé avec ladite émulsion selon un pourcentage variant de 20 à 80%, de préférence 50% en poids.

3. Procédé selon l'une ou plusieurs des revendications précédentes, **caractérisé en ce que** ladite émulsion comprend entre 1,0 % et 22 %, de préférence 15% en poids, de ladite au moins une silicone volatile.

4. Procédé selon l'une ou plusieurs des revendications précédentes, **caractérisé en ce que** ladite émulsion comprend entre 5% et 40 %, de préférence 15% en poids, de ladite au moins une silicone non volatile.

5. Procédé selon l'une ou plusieurs des revendications précédentes, **caractérisé en ce que** ladite émulsion comprend entre 0,3% et 5 %, de préférence 0,7% en poids, de ladite au moins une silicone émulsionnante.

6. Procédé selon l'une ou plusieurs des revendications précédentes, **caractérisé en ce que** ladite émulsion comprend entre 0,2% et 5%, de préférence 3% en poids, d'un agent gélifiant.
